(19) Europäisches Patentamt
European Patent Office
Office européen des brevets

(11) Veröffentlichungsnummer: **0 624 570 A2**

# (12) EUROPÄISCHE PATENTANMELDUNG

(21) Anmeldenummer: **94106895.9**

(22) Anmeldetag: **03.05.94**

(51) Int. Cl.5: **C07C 251/48**, C07C 249/08

(30) Priorität: **14.05.93 DE 4316187**
**21.06.93 DE 4320499**

(43) Veröffentlichungstag der Anmeldung:
**17.11.94 Patentblatt 94/46**

(84) Benannte Vertragsstaaten:
**BE CH DE ES FR GB IT LI NL**

(71) Anmelder: **BAYER AG**

**D-51368 Leverkusen (DE)**

(72) Erfinder: **Kuhnt, Dietmar Dr.**
**Körnerstrasse 5**
**D-51373 Leverkusen (DE)**
Erfinder: **Gayer, Herbert Dr.**
**Sandstrasse 66**
**D-40789 Monheim (DE)**
Erfinder: **Gerdes, Peter Dr.**
**Waldstrasse 75**
**D-52080 Aachen (DE)**

(54) **Verfahren zur Herstellung von 2-Oximinoessigsäurederivaten.**

(57) Beschrieben wird ein neues Verfahren und neue Zwischenprodukte zur Herstellung von 2-Oximinoessigsäurederivaten der Formel (I)

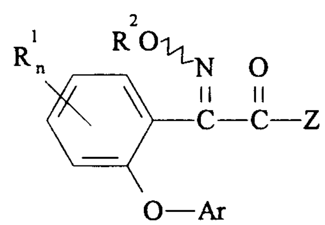

(I)

worin $R^1$, $R^2$, n, Z und Ar die in der Beschreibung angegebene Bedeutung haben und welche als Schädlingsbekämpfungsmittel verwendet werden.

EP 0 624 570 A2

Die Erfindung betrifft ein neues Verfahren und neue Zwischenprodukte zur Herstellung von 2-Oximinoessigsäurederivaten, welche als Schädlingsbekämpfungsmittel verwendet werden.

Es ist bekannt, daß man fungizide 2-Methoximino-2-phenylessigsäurederivate auf verschiedenen in der Regel vielstufigen Synthesewegen erhält, wobei im allgemeinen die entsprechenden 2-Oxo-2-phenylessigsäurederivate als zentrale Zwischenprodukte auftreten (vergl. z.B. EP 398 692 und EP 468 775). Der Nachteil aller dieser Synthesewege besteht darin, daß diese 2-Oxo-2-phenylessigsäurederivate durch Umsetzung mit metallorganischen Verbindungen (z.B. n-Butyllithium) hergestellt werden, eine Methode die technisch aufwendig zu realisieren ist. Auch die Ausbeuten der bisher bekannten Verfahren sind in der Regel unbefriedigend.

Es wurde gefunden, daß man fungizide 2-Oximinoessigsäurederivate der allgemeinen Formel (I),

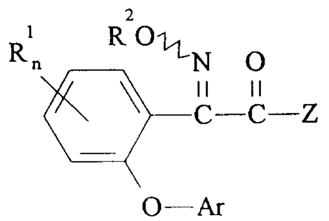

(I)

in welcher

$R^1$ für Alkyl oder Alkoxy steht,
$R^2$ für Alkyl steht,
Ar für gegebenenfalls substituiertes Aryl oder Heteroaryl steht,
Z für einen Rest der Formel $-O-R^3$ oder $-NR^4R^5$ steht und
n für eine Zahl 0, 1, 2, 3 oder 4 steht, wobei
$R^3$ für Alkyl steht und
$R^4$ und $R^5$ unabhängig voneinander jeweils für Wasserstoff, Alkyl, Halogenalkyl oder Alkoxy stehen,

erhält, wenn man 2-Oxo-2-phenyl-essigsäurederivate der Formel (II),

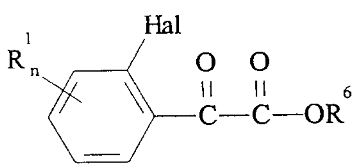

(II)

in welcher

Hal für Halogen steht,
$R^6$ für Wasserstoff oder Alkyl steht und
$R^1$ und n die oben angegebene Bedeutung haben,

zunächst in einer 1. Stufe mit Alkoholen der Formel (IIIa),

$R^7$-OH (IIIa)

in welcher

$R^7$ für Alkyl steht,

oder mit Diolen der Formel (IIIb),

HO-$R^7$-$R^8$-OH (IIIb)

in welcher

$R^7$ und $R^8$ gemeinsam für einen zweifach verknüpften Alkandiylrest stehen,

gegebenenfalls in Gegenwart eines Verdünnungsmittels und gegebenenfalls in Gegenwart eines Reaktionshilfsmittels umsetzt und in einer anschließenden 2. Stufe die so erhältlichen 2-Aryl-2,2-dialkoxy-essigsäurederivate der Formel (IVa),

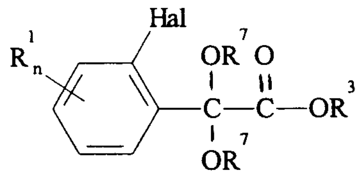

(IVa-1)

bzw. 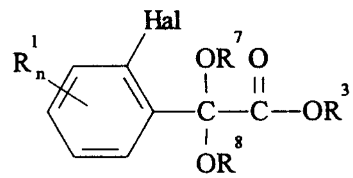

(IVa-2)

in welcher

$R^1$, $R^3$, $R^7$, $R^8$, Hal und n die oben angegebene Bedeutung haben,

mit Hydroxyverbindungen der Formel (V),

Ar-OH (V)

in welcher

Ar die oben angegebene Bedeutung hat,

gegebenenfalls in Gegenwart eines Verdünnungsmittels und gegebenenfalls in Gegenwart eines Reaktionshilfsmittels umsetzt und anschließend die so erhältlichen 2-Aryl-2,2-dialkoxy-essigsäurederivate der Formel (VIa),

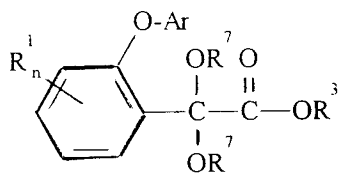

(VIa-1)

bzw. 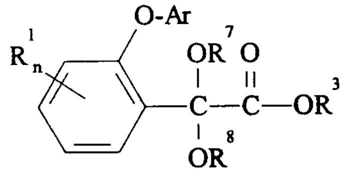

(VIa-2)

in welcher

$R^1$, $R^3$, $R^7$, $R^8$, Ar und n die oben angegebene Bedeutung haben,

in einer darauffolgenden 3. Stufe mit Hydroxylaminderivaten der Formel (VII),

$R^2$-O-$NH_2$ (VII)

in welcher

$R^2$ die oben angegebene Bedeutung hat,

oder mit deren Hydrohalogenidsalzen gegebenenfalls in Gegenwart eines Verdünnungsmittels und gegebenenfalls in Gegenwart eines Reaktionshilfsmittels zu den 2-Oximinoessigsäurederivaten der Formel (Ia),

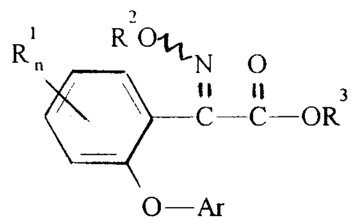

(Ia)

in welcher

$R^1$, $R^2$, $R^3$, Ar und n die oben angegebene Bedeutung haben,

umsetzt.

Dabei können gegebenenfalls entweder die in der ersten Stufe erhältlichen 2-Aryl-2,2-dialkoxy-essigsäurederivate der Formel (IVa) oder die in der zweiten Stufe erhältlichen 2-Aryl-2,2-dialkoxy-essigsäurederivate der Formel (VIa) oder die in der dritten Stufe erhältlichen 2-Oximinoessigsäurederivaten der Formel (Ia) in einer zwischengeschalteten Reaktion bzw. in einer Folgereaktion jeweils mit Aminen der Formel (VIII),

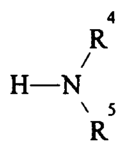

(VIII)

in welcher

$R^5$ und $R^6$ die oben angegebene Bedeutung haben,

gegebenenfalls in Gegenwart eines Verdünnungsmittels und gegebenenfalls Gegenwart eines Restionshilfsmittels umgesetzt werden. In diesem Fall werden als Ausgangsverbindungen für die 2. bzw. 3. Stufe des erfindungsgemäßen Herstellungsverfahrens die jeweiligen entsprechen Amide der Formeln (IVb),

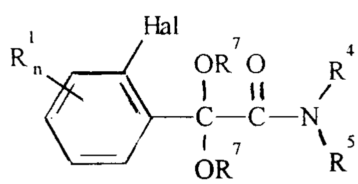

(IVb-1)

bzw.

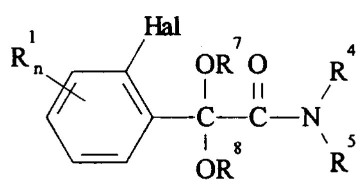

(IVb-2)

in welcher

$R^1$, $R^4$, $R^5$, $R^7$, $R^8$, Hal und n die oben angegebene Bedeutung haben,

bzw. (VIb),

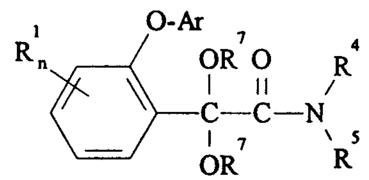

(VIb-1)

bzw. 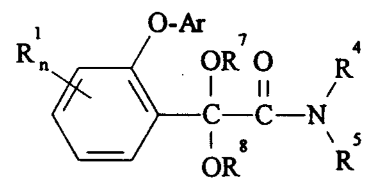

(VIb-2)

in welcher

$R^1$, $R^4$, $R^5$, $R^7$, $R^8$, Ar und n die oben angegebene Bedeutung haben,

eingesetzt.

Überraschenderweise erhält man bei der Durchführung des erfindungsgemäßen Verfahrens die erwünschten fungizid wirksamen 2-Oximinoessigsäurederivate, der Formel (I) in glatter Reaktion und in besserer Ausbeute über alle Stufen als nach den aus dem Stand der Technik bekannten Verfahren.

Es ist darüberhinaus auch als besonders überraschend anzusehen, daß bei der 2. Stufe des erfindungsgemäßen Verfahrens die Umsetzung der 2-Halogengruppierung im Phenylteil der Zwischenprodukte der Formeln (IVa) und (IVb) mit Phenolen der Formel (V) bei Vorhandensein einer Ketalstruktur in Nachbarstellung zum Phenylring in glatter Reaktion verläuft, da die vergleichbare Umsetzung entsprechender 2-Oxo-2-phenyl-essigsäurederivate oder entsprechender 2-Alkoximino-2-phenyl-essigsäurederivate mit Phenolen der Formel (V) zur weitgehenden Zersetzung der Ausgangsprodukte führt ohne daß nennenswerte Mengen an gewünschtem Endprodukt erhalten werden.

Ein besonderer Vorteil des erfindungsgemäßen Verfahrens ist dabei, daß von leicht zugänglichen Vorprodukten ausgegangen werden kann und der Einsatz von großtechnisch schwer handhabbaren metallorganischen Verbindungen, wie beispielsweise Butyllithium, vermieden wird.

Die erfindungsgemäß herstellbaren 2-Oximinoessigsäurederivate sind durch die Formel (I) allgemein definiert. Bevorzugt herstellbar sind Verbindungen der Formel (I), bei welchen

$R^1$ für geradkettiges oder verzweigtes Alkyl mit 1 bis 6 Kohlenstoffatomen oder für geradkettiges oder verzweigtes Alkoxy mit 1 bis 6 Kohlenstoffatomen steht,

$R^2$ für geradkettiges oder verzweigtes Alkyl mit 1 bis 6 Kohlenstoffatomen steht.

Ar für jeweils gegebenenfalls einfach oder mehrfach, gleich oder verschieden substituiertes Aryl mit 6 bis 10 Kohlenstoffatomen oder Heteroaryl mit 2 bis 9 Kohlenstoffatomen und 1 bis 4 gleichen oder verschiedenen Heteroatomen - insbesondere Stickstoff Sauerstoff und/oder Schwefel - steht, wobei als Substituenten jeweils infrage kommen:

Halogen, Cyano, Nitro, jeweils geradkettiges oder verzweigtes Alkyl, Alkoxy, Alkylthio mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Alkinyl oder Alkinyloxy mit jeweils 2 bis 6 Kohlenstoffatomen, jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Halogenalkinyl oder Halogenalkinyloxy mit jeweils 2 bis 6 Kohlenstoffatomen und 1 bis 13 gleichen oder verschiedenen Halogenatomen, jeweils geradkettiges oder verzweigtes Alkoxycarbonyl, Hydroximinoalkyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit

1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - substituiertes zweifach verknüpftes Dioxyalkylen mit 1 bis 5 Kohlenstoffatomen oder jeweils gegebenenfalls im Phenylteil einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Benzyl, Benzyloxy, Phenylethyl oder Phenylethyloxy,

Z für einen Rest der Formel $-O-R^3$ oder $-NR^4R^5$ steht und

n für eine Zahl 0, 1, 2, 3 oder 4 steht, wobei

$R^3$ für geradkettiges oder verzweigtes Alkyl mit 1 bis 6 Kohlenstoffatomen steht und

$R^4$ und $R^5$ unabhängig voneinander jeweils für Wasserstoff für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen steht oder für geradkettiges oder verzweigtes Alkoxy mit 1 bis 8 Kohlenstoffatomen stehen.

Besonders bevorzugt herstellbar sind Verbindungen der Formel (I), bei welchen

$R^1$ für geradkettiges oder verzweigtes Alkyl mit 1 bis 4 Kohlenstoffatomen oder für geradkettiges oder verzweigtes Alkoxy mit 1 bis 4 Kohlenstoffatomen steht,

$R^2$ für geradkettiges oder verzweigtes Alkyl mit 1 bis 4 Kohlenstoffatomen steht,

Ar für jeweils gegebenenfalls einfach bis fünffach, gleich oder verschieden substituiertes Aryl mit 6 oder 10 Kohlenstoffatomen oder Heteroaryl mit 2 bis 9 Kohlenstoffatomen und 1 bis 3 gleichen oder verschiedenen Heteroatomen - insbesondere Stickstoff Sauerstoff und/oder Schwefel - steht, wobei als Substituenten jeweils infrage kommen:

Halogen, Cyano, Nitro, jeweils geradkettiges oder verzweigtes Alkyl, Alkoxy, Alkylthio mit jeweils 1 bis 4 Kohlenstoffatomen, jeweils geradkettiges oder verzweigtes Alkenyl oder Alkenyloxy mit jeweils 2 bis 5 Kohlenstoffatomen, jeweils geradkettiges oder verzweigtes Alkinyl oder Alkinyloxy mit jeweils 2 bis 5 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Halogenalkenyl oder Halogenalkenyloxy mit jeweils 2 bis 5 Kohlenstoffatomen und 1 bis 5 gleichen oder verschiedenen Halogenatomen, jeweils geradkettiges oder verzweigtes Halogenalkinyl oder Halogenalkinyloxy mit jeweils 2 bis 5 Kohlenstoffatomen und 1 bis 5 gleichen oder verschiedenen Halogenatomen, jeweils geradkettiges oder verzweigtes Alkoxycarbonyl, Hydroximinoalkyl oder Alkoximinoalkyl mit jeweils 1 bis 4 Kohlenstoffatomen in den einzelnen Alkylteilen, gegebenenfalls einfach bis vierfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - substituiertes zweifach verknüpftes Dioxyalkylen mit 1 bis 3 Kohlenstoffatomen oder jeweils gegebenenfalls im Phenylteil einfach bis fünffach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 3 Kohlenstoffatomen und 1 bis 7 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 3 Kohlenstoffatomen und 1 bis 7 gleichen oder verschiedenen Halogenatomen substituiertes Phenyl, Phenoxy, Benzyl, Benzyloxy, Phenylethyl oder Phenylethyloxy,

Z für einen Rest der Formel $-O-R^3$ oder $-NR^4R^5$ steht und

n für eine zahl 0, 1, 2 oder 3 steht, wobei

$R^3$ für geradkettiges oder verzweigtes Alkyl mit 1 bis 4 Kohlenstoffatomen steht und

$R^4$ und $R^5$ unabhängig voneinander jeweils für Wasserstoff für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen steht oder für geradkettiges oder verzweigtes Alkoxy mit 1 bis 6 Kohlenstoffatomen stehen.

Ganz besonders bevorzugt herstellbar sind Verbindungen der Formel (I), bei welchen

$R^1$ für Methyl, Methoxy, Ethyl oder Ethoxy steht,

$R^2$ für Methyl oder Ethyl steht,

Ar für gegebenenfalls einfach bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Allyl, Butenyl, Allyloxy, Butenyloxy, Trifluormethyl, Trifluormethoxy, Difluormethoxy, Trifluormethylthio, Difluorchlormethylthio, Methoxycarbonyl, Ethoxycarbonyl, Hydroximinomethyl, Hydroximinoethyl, Methoximinomethyl, Methoximinoethyl Ethoximinomethyl, Ethoximinoethyl, gegebenenfalls einfach bis vierfach, gleich oder verschieden durch Fluor, Chlor, Brom, Methyl, Ethyl und/oder Trifluormethyl substituiertes zweifach verknüpftes Dioxymethylen oder Dioxyethylen oder jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy,

Z für einen Rest der Formel $-O-R^3$ oder $-NR^4R^5$ steht und

n für eine Zahl 0,1, oder 2 steht, wobei

$R^3$ für Methyl oder Ethyl steht und

$R^4$ und $R^5$ unabhängig voneinander jeweils für Wasserstoff für geradkettiges oder verzweigtes Alkyl mit 1 bis 4 Kohlenstoffatomen, für Halogenalkyl mit 1 bis 4 Kohlenstoffatomen und 1 bis 5 gleichen oder verschiedenen Halogenatomen steht oder für geradkettiges oder verzweigtes Alkoxy mit 1 bis 4 Kohlenstoffatomen stehen.

Verwendet man beispielsweise 2-Chlorphenylglyoxylsäuremethylester und Methanol als Ausgangsstoffe, so läßt sich der Reaktionsablauf des erfindungsgemäßen Verfahrens durch das folgende Formelschema darstellen:

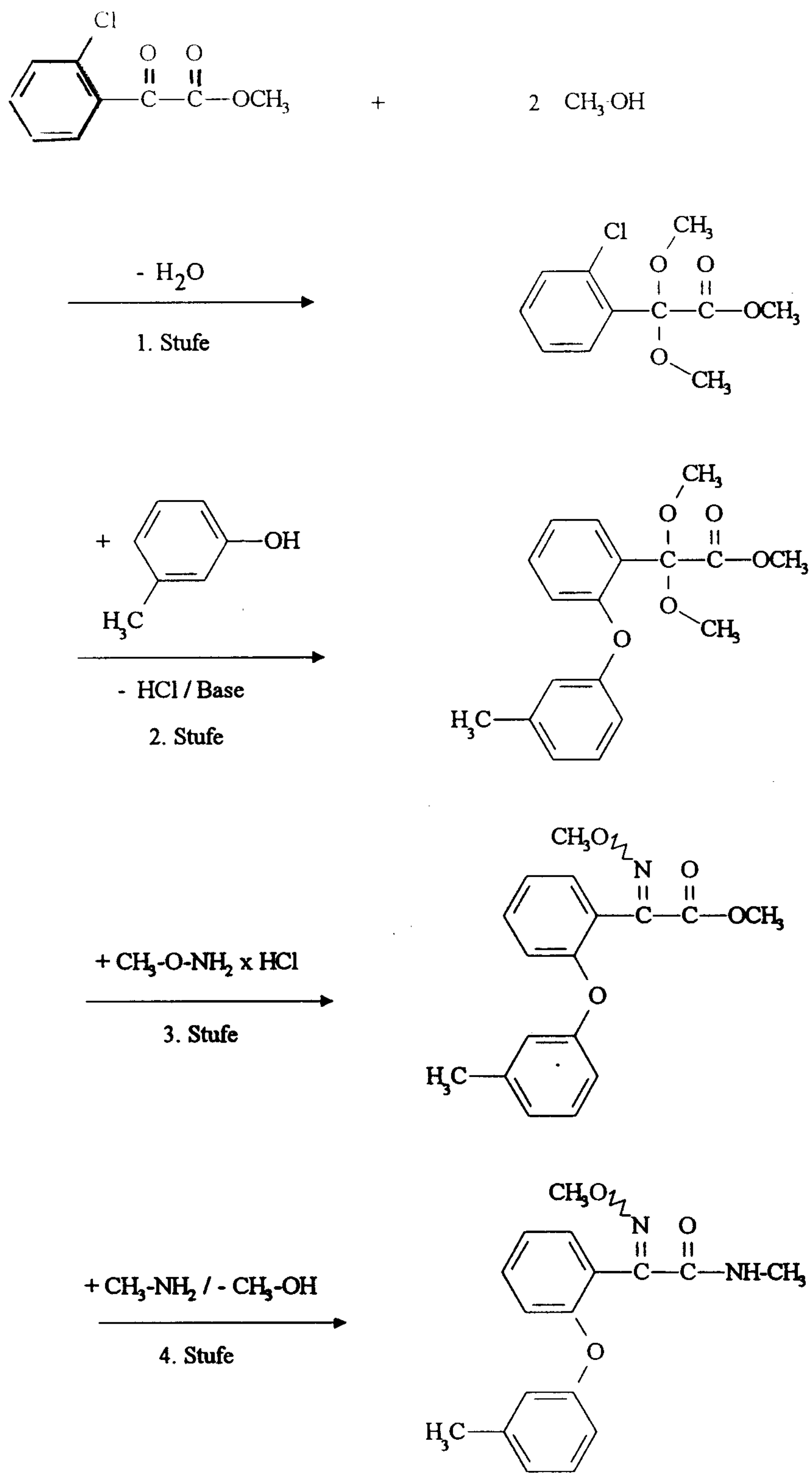


Die zur Durchführung des erfindungsgemäßen Verfahrens in der ersten Stufe als Ausgangsverbindungen benötigten 2-Oxo-2-phenyl-essigsäurederivate sind durch die Formel (II) allgemein definiert. In dieser Formel (II) stehen $R^1$ und n vorzugsweise für diejenigen Reste und Indices, die bereits im Zusammenhang mit der Beschreibung der erfindungsgemäß herstellbaren Verbindungen der Formel (I) als bevorzugt für diesen Substituenten und diesen Index genannt wurden. $R^6$ steht vorzugsweise für Wasserstoff oder für geradkettiges oder verzweigtes Alkyl mit 1 bis 4 Kohlenstoffatomen, insbesondere für Wasserstoff Methyl

oder Ethyl. Hal steht vorzugsweise für Fluor, Chlor, Brom oder Iod, insbesondere für Chlor oder Brom.

2-Oxo-2-phenyl-essigsäurederivate der Formel (II) sind bekannt oder erhältlich in Analogie zu bekannten Verfahren (vergl. z.B. FR 15 56 822; J. Sci. Soc. Thailand 8, 215-223 [1982]).

Die zur Durchführung des erfindungsgemäßen Verfahrens in der ersten Stufe weiterhin als Ausgangsstoffe benötigten Alkohole oder Diole sind durch die Formeln (IIIa) bzw. (IIIb) allgemein definiert. In diesen Formeln (IIIa) und (IIIb) stehen $R^7$ und $R^8$ vorzugsweise entweder jeweils für geradkettiges oder verzweigtes Alkyl mit 1 bis 6, insbesondere 1 bis 4 Kohlenstoffatomen oder gemeinsam für einen zweifach verknüpften Alkandiylrest mit 1 bis 6 Kohlenstoffatomen.

Die Alkohole oder Diole der Formeln (IIIa) und (IIIb) sind allgemein bekannte Verbindungen der organischen Chemie.

Die zur Durchführung des erfindungsgemäßen Verfahrens in der zweiten Stufe weiterhin als Ausgangsstoffe benötigten Hydroxyverbindungen sind durch die Formel (V) allgemein definiert. In dieser Formel (V) steht Ar vorzugsweise für diejenigen Reste, die bereits im Zusammenhang mit der Beschreibung der erfindungsgemäß herstellbaren Verbindungen der Formel (I) als bevorzugt für diese Substituenten genannt wurden.

Die Hydroxyverbindungen der Formel (V) sind ebenfalls allgemein bekannte Verbindungen der organischen Chemie.

Die zur Durchführung des erfindungsgemäßen Verfahrens in der dritten Stufe weiterhin als Ausgangsstoffe benötigten Hydroxylaminderivate sind durch die Formel (VII) allgemein definiert. In dieser Formel (VII) steht $R^2$ vorzugsweise für diejenigen Reste, die bereits im Zusammenhang mit der Beschreibung der erfindungsgemäß herstellbaren Verbindungen der Formel (I) als bevorzugt für diese Substituenten genannt wurden. Als deren Hydrohalogenide kommen insbesondere die Hydrochloride und Hydrobromide infrage.

Die Hydroxylaminderivate der Formel (VII) und deren Hydrohalogenide sind ebenfalls allgemein bekannte Verbindungen der organischen Chemie.

Die zur Durchführung der erfindungsgemäßen Verfahrens gegebenenfalls weiterhin als Ausgangsstoffe benötigten Amine sind durch die Formel (VIII) allgemein definiert. In dieser Formel (VIII) stehen $R^4$ und $R^5$ vorzugsweise für diejenigen Reste, die bereits im Zusammenhang mit der Beschreibung der erfindungsgemäß herstellbaren Verbindungen der Formel (I) als bevorzugt für diese Substituenten genannt wurden.

Die Amine der Formel (VIII) sind ebenfalls allgemein bekannte Verbindungen der organischen Chemie.

Die bei der Durchführung des erfindungsgemäßen Verfahrens als Zwischenprodukte auftretenden 2-Aryl-2,2-dialkoxy-essigsäurederivate der Formel (IX),

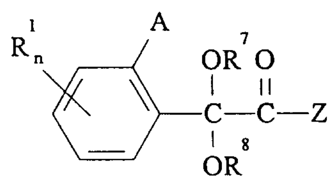

(IX)

in welcher

| | |
|---|---|
| $R^1$ | für Alkyl oder Alkoxy steht, |
| $R^7$ und $R^8$ | entweder gleich sind und jeweils für Alkyl stehen oder gemeinsam für einen zweifach verknüpften Alkandiylrest stehen, |
| A | für Halogen oder für gegebenenfalls substituiertes Aryloxy oder Heteroaryloxy steht, |
| Z | für einen Rest der Formel $-O-R^3$ oder $-NR^4R^5$ steht und |
| n | für eine Zahl 0, 1, 2, 3 oder 4 steht, wobei |
| $R^3$ | für Alkyl steht und |
| $R^4$ und $R^5$ | unabhängig voneinander jeweils für Wasserstoff Alkyl, Halogenalkyl oder Alkoxy stehen, |

entsprechen den Verbindugnen der Formel (IV) und (VI) und sind noch nicht bekannt und ebenfalls Gegenstand der Erfindung.

Bevorzugt sind Verbindungen der Formel (IX), bei welchen

| | |
|---|---|
| $R^1$ | für geradkettiges oder verzweigtes Alkyl mit 1 bis 6 Kohlenstoffatomen oder für geradkettiges oder verzweigtes Alkoxy mit 1 bis 6 Kohlenstoffatomen steht, |
| $R^7$ und $R^8$ | entweder gleich sind und für geradkettiges oder verzweigtes Alkyl mit 1 bis 6 Kohlenstoffatomen stehen oder gemeinsam für einen zweifach verknüpften Alkandiylrest mit 1 bis 6 Kohlenstoffatomen stehen, |
| A | für Fluor, Chlor, Brom, Iod oder für jeweils gegebenenfalls einfach oder mehrfach, gleich |

oder verschieden substituiertes Aryloxy mit 6 bis 10 Kohlenstoffatomen oder Heteroaryloxy mit 2 bis 9 Kohlenstoffatomen und 1 bis 4 gleichen oder verschiedenen Heteroatomen - insbesondere Stickstoff Sauerstoff und/oder Schwefel - steht, wobei als Substituenten jeweils infrage kommen:

Halogen, Cyano, Nitro, jeweils geradkettiges oder verzweigtes Alkyl, Alkoxy, Alkylthio mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Alkinyl oder Alkinyloxy mit jeweils 2 bis 6 Kohlenstoffatomen, jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Halogenalkinyl oder Halogenalkinyloxy mit jeweils 2 bis 6 Kohlenstoffatomen und 1 bis 13 gleichen oder verschiedenen Halogenatomen, jeweils geradkettiges oder verzweigtes Alkoxycarbonyl, Hydroximinoalkyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - substituiertes zweifach verknüpftes Dioxyalkylen mit 1 bis 5 Kohlenstoffatomen oder jeweils gegebenenfalls im Phenylteil einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Benzyl, Benzyloxy, Phenylethyl oder Phenylethyloxy,

Z für einen Rest der Formel -O-$R^3$ oder -$NR^4R^5$ steht und

n für eine Zahl 0, 1, 2, 3 oder 4 steht, wobei

$R^3$ für geradkettiges oder verzweigtes Alkyl mit 1 bis 6 Kohlenstoffatomen steht und

$R^4$ und $R^5$ unabhängig voneinander jeweils für Wasserstoff, für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen steht oder für geradkettiges oder verzweigtes Alkoxy mit 1 bis 8 Kohlenstoffatomen stehen.

Besonders bevorzugt sind Verbindungen der Formel (IX), bei welchen

$R^1$ für geradkettiges oder verzweigtes Alkyl mit 1 bis 4 Kohlenstoffatomen oder für geradkettiges oder verzweigtes Alkoxy mit 1 bis 4 Kohlenstoffatomen steht,

$R^7$ und $R^8$ entweder gleich sind und für geradkettiges oder verzweigtes Alkyl mit 1 bis 4 Kohlenstoffatomen stehen oder gemeinsam für einen zweifach verknüpften Alkandiylrest mit 2 bis 5 Kohlenstoffatomen stehen,

A für Fluor, Chlor, Brom oder für jeweils gegebenenfalls einfach bis fünffach, gleich oder verschieden substituiertes Aryloxy mit 6 oder 10 Kohlenstoffatomen oder Heteroaryloxy mit 2 bis 9 Kohlenstoffatomen und 1 bis 3 gleichen oder verschiedenen Heteroatomen - insbesondere Stickstoff, Sauerstoff und/oder Schwefel - steht, wobei als Substituenten jeweils infrage kommen:

Halogen, Cyano, Nitro, jeweils geradkettiges oder verzweigtes Alkyl, Alkoxy, Alkylthio mit jeweils 1 bis 4 Kohlenstoffatomen, jeweils geradkettiges oder verzweigtes Alkenyl oder Alkenyloxy mit jeweils 2 bis 5 Kohlenstoffatomen, jeweils geradkettiges oder verzweigtes Alkinyl oder Alkinyloxy mit jeweils 2 bis 5 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Halogenalkenyl oder Halogenalkenyloxy mit jeweils 2 bis 5 Kohlenstoffatomen und 1 bis 5 gleichen oder verschiedenen Halogenatomen, jeweils geradkettiges oder verzweigtes Halogenalkinyl oder Halogenalkinyloxy mit jeweils 2 bis 5 Kohlenstoffatomen und 1 bis 5 gleichen oder verschiedenen Halogenatomen, jeweils geradkettiges oder verzweigtes Alkoxycarbonyl, Hydroximinoalkyl oder Alkoximinoalkyl mit jeweils 1 bis 4 Kohlenstoffatomen in den einzelnen Alkylteilen, gegebenenfalls einfach bis vierfach, gleich

oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - substituiertes zweifach verknüpftes Dioxyalkylen mit 1 bis 3 Kohlenstoffatomen oder jeweils gegebenenfalls im Phenylteil einfach bis fünffach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 3 Kohlenstoffatomen und 1 bis 7 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 3 Kohlenstoffatomen und 1 bis 7 gleichen oder verschiedenen Halogenatomen substituiertes Phenyl, Phenoxy, Benzyl, Benzyloxy, Phenylethyl oder Phenylethyloxy,

Z für einen Rest der Formel -O-$R^3$ oder -$NR^4R^5$ steht und

n für eine zahl 0, 1, 2 oder 3 steht, wobei

$R^3$ für geradkettiges oder verzweigtes Alkyl mit 1 bis 4 Kohlenstoffatomen steht und

$R^4$ und $R^5$ unabhängig voneinander jeweils für Wasserstoff, für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen steht oder für geradkettiges oder verzweigtes Alkoxy mit 1 bis 6 Kohlenstoffatomen stehen.

Ganz besonders bevorzugt sind Verbindungen der Formel (I), bei welchen

$R^1$ für Methyl, Methoxy, Ethyl oder Ethoxy steht,

$R^7$ und $R^8$ entweder gleich sind und für Methyl oder Ethyl stehen oder gemeinsam für einen jeweils zweifach verknüpften Ethan-1,2-diylrest oder Propan-1,3-diylrest stehen,

A für Chlor, Brom oder für gegebenenfalls einfach bis dreifach, gleich oder verschieden substituiertes Phenoxy steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Allyl, Butenyl, Allyloxy, Butenyloxy, Trifluormethyl, Trifluormethoxy, Difluormethoxy, Trifluormethylthio, Difluorchlormethylthio, Methoxycarbonyl, Ethoxycarbonyl, Hydroximinomethyl, Hydroximinoethyl, Methoximinomethyl, Methoximinoethyl, Ethoximinomethyl, Ethoximinoethyl, gegebenenfalls einfach bis vierfach, gleich oder verschieden durch Fluor, Chlor, Brom, Methyl, Ethyl und/oder Trifluormethyl substituiertes zweifach verknüpftes Dioxymethylen oder Dioxyethylen oder jeweils gegebenenfalls einbis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy,

Z für einen Rest der Formel -O-$R^3$ oder -$NR^4R^5$ steht und

n für eine Zahl 0,1, oder 2 steht, wobei

$R^3$ für Methyl oder Ethyl steht und

$R^4$ und $R^5$ unabhängig voneinander jeweils für Wasserstoff für geradkettiges oder verzweigtes Alkyl mit 1 bis 4 Kohlenstoffatomen, für Halogenalkyl mit 1 bis 4 Kohlenstoffatomen und 1 bis 5 gleichen oder verschiedenen Halogenatomen steht oder für geradkettiges oder verzweigtes Alkoxy mit 1 bis 4 Kohlenstoffatomen stehen.

Im einzelnen seien außer den bei den Herstellungsbeispielen genannten Verbindungen die folgenden 2-Aryl-2,2-dialkoxy-essigsäurederivate allgemeinen Formel (IX) genannt:

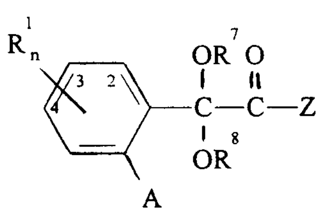

(IX)

| A | $R^1$ | $R^7$ | $R^8$ | n | Z |
|---|---|---|---|---|---|
| Cl | H | $C_2H_5$ | $C_2H_5$ | 0 | $-O-C_2H_5$ |
| Br | H | $C_2H_5$ | $C_2H_5$ | 0 | $-O-C_2H_5$ |
| Cl | H | $CH_3$ | $CH_3$ | 0 | $-NH-CH_3$ |
| Cl | H | $CH_3$ | $CH_3$ | 0 | $-NH-C_2H_5$ |
| Cl | H | $C_2H_5$ | $C_2H_5$ | 0 | $-NH-CH_3$ |
| Br | H | $CH_3$ | $CH_3$ | 0 | $-NH-CH_3$ |
| Br | H | $C_2H_5$ | $C_2H_5$ | 0 | $-NH-CH_3$ |
| Cl | $4-CH_3$ | $CH_3$ | $CH_3$ | 1 | $-O-CH_3$ |
| Br | $4-CH_3$ | $CH_3$ | $CH_3$ | 1 | $-O-CH_3$ |
| $-O-C_6H_5$ | H | $C_2H_5$ | $C_2H_5$ | 0 | $-O-C_2H_5$ |
| 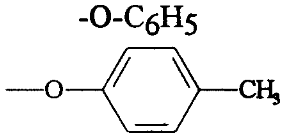 | H | $CH_3$ | $CH_3$ | 0 | $-O-CH_3$ |
| 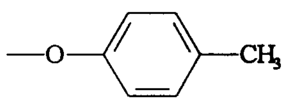 | H | $CH_3$ | $CH_3$ | 0 | $-NH-CH_3$ |

Als Verdünnungsmittel zur Durchführung der 1. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran, Ethylenglykoldimethyl-oder -diethylether, Diethylenglykoldimethyl- oder -diethylether, Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid. Es ist auch möglich, die als Reaktionspartner infrage kommenden Alkohole oder Diole der Formeln (IIIa) bzw. (IIIb) in entsprechendem Überschuß gleichzeitig als Verdünnungsmittel einzusetzen.

Die 1. Stufe des erfindungsgemäßen Verfahrens wird vorzugsweise in Gegenwart eines geeigneten Reaktionshilfsmittels durchgeführt. Als solche kommen alle üblichen wasserentziehenden Mittel infrage. Hierzu gehören insbesondere Orthoester, wie beispielsweise Ortho-Ameisensäuremethylester oder Ortho-Ameisensäureethylester, Sulfonsäuren, wie beispielsweise Methansulfonsäure oder p-Toluolsulfonsäure, Mineralsäuren, wie Salzsäure oder Schwefelsäure, saure Ionenaustauscher oder Molekularsieb. Es ist auch möglich freiwerdenden Reaktionswasser durch azeotrope Destillation aus dem Reaktionsgemisch zu entfernen.

Die Reaktionstemperaturen können bei der Durchführung der 1. Stufe des erfindungsgemäßen Verfahrens in einem größeren Bereich variiert werden. Im allgemeinen arbeitet man bei Temperaturen von 0 °C bis + 150 °C, vorzugsweise bei Temperaturen von 20 °C bis +120 °C.

Die 1. Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der 1. Stufe des erfindungsgemäßen Verfahrens setzt man pro Mol an 2-Oxo-2-phenyl-essigsäurederivat der Formel (II) im allgemeinen 1,0 bis 50,0 Mol, vorzugsweise 1,0 bis 20,0 Mol an Alkohol oder Diol der Formeln (IIIa) bzw. (IIIb) und gegebenenfalls 1,0 bis 10,0 Mol, vorzugsweise 1,0 bis 5,0 Mol an Reaktionshilfsmittel ein. Die Reaktionsdurchführung, Aufarbeitung und Isolierung der Reaktionsprodukte erfolgt nach allgemein bekannten Verfahren (vergleiche hierzu auch die Herstellungsbeispiele).

Als Verdünnungsmittel zur Durchführung der 2. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran oder Ethylenglykoldimethyl-

oder -diethylether, Diethylenglykoldimethyl- oder - diethylether; Ketone, wie Aceton, Butanon oder Methylisobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid, Sulfolan oder Alkohole, wie Methanol, Ethanol, n- oder i-Propanol, Ethylenglykolmonomethylether, Ethylenglykolmonoethylether, Diethylenglykolmonomethylether, Diethylenglykolmonoethylether.

Die 2. Stufe des erfindungsgemäßen Verfahrens wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die 2. Stufe des erfindungsgemäßen Verfahrens wird außerdem vorzugsweise in Gegenwart eines geeigneten Katalysators durchgeführt. Als solche kommen insbesondere Kupfer-(I)-Salze, wie beispielsweise Kupfer-(I)-chlorid infrage. Hierbei kann der Zusatz von katalytischen Mengen eines geeigneten Phasentransferkatalysators, wie beispielsweise 15-Krone-5, 18-Krone-6 oder Tris-[2-(2-methoxyethoxy)-ethyl]-amin von Vorteil sein.

Die Reaktionstemperaturen können bei der Durchführung der 2. Stufe des erfindungsgemäßen Verfahrens in einem größeren Bereich variiert werden. Im allgemeinen arbeitet man bei Temperaturen von 50 °C bis +250 °C, vorzugsweise bei Temperaturen von 120 °C bis +200 °C.

Die 2. Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der 2. Stufe des erfindungsgemäßen Verfahrens setzt man pro Mol an 2-Aryl-2,2-dialkoxy-essigsäurederivat der Formel (IV) im allgemeinen 1,0 bis 1,5 Mol, vorzugsweise 1,0 bis 1,2 Mol an Hydroxyverbindung der Formel (V), 1,0 bis 1,5 Mol, vorzugsweise 1,0 bis 1,2 Mol an basischem Reaktionshilfsmittel und gegebenenfalls 0,0001 bis 1,0 Mol, vorzugsweise 0,001 bis 0,1 Mol an Kupfer-(I)-salz-Katalysator ein. Die Reaktionsdurchführung, Aufarbeitung und Isolierung der Reaktionsprodukte erfolgt in Analogie zu bekannten Verfahren (vergleiche hierzu auch die Herstellungsbeispiele).

Als Verdünnungsmittel zur Durchführung der 3. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die 3. Stufe des erfindungsgemäßen Verfahrens wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 3 Stufe des erfindungsgemäßen Verfahrens in einem größeren Bereich variiert werden. Im allgemeinen arbeitet man bei Temperaturen von 0 °C bis +150 °C, vorzugsweise bei Temperaturen von 20 °C bis +120 °C.

Die 3. stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der 3. Stufe des erfindungsgemäßen Verfahrens setzt man pro Mol an 2-Aryl-2,2-dialkoxy-essigsäurederivat der Formel (VI) im allgemeinen 1,0 bis 3,0 Mol, vorzugsweise 1,0 bis 2,0 Mol an Hydroxylamin der Formel (VII) bzw. eines entsprechenden Hydrohalogenids und gegebenenfalls 1,0 bis 6,0

Mol, vorzugsweise 1,0 bis 3,0 Mol an Reaktionshilfsmittel ein. Die Reaktionsdurchführung, Aufarbeitung und Isolierung der Reaktionsprodukte erfolgt nach bekannten Verfahren.

Als Verdünnungsmittel zur Durchführung der gegebenenfalls durchzuführenden 4. Stufe des erfindungsgemäßen Verfahrens (Amidierungsreaktion) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril: Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfolan oder Alkohole, wie Methanol, Ethanol, n- oder i-Propanol, Ethylenglykolmonomethylether, Ethylenglykolmonoethylether, Diethylenglykolmonomethylether, Diethylenglykolmonoethylether.

Es ist auch möglich, das als Reaktionspartner verwendete Amin der Formel (VIII) in einem entsprechenden Überschuß gleichzeitig als Verdünnungsmittel einzusetzen.

Die gegebenenfalls durchzuführende 4. Stufe des erfindungsgemäßen Verfahrens (Amidierungsreaktion) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (VIII) in einem entsprechenden Überschuß gleichzeitig als Reaktionshilfmittel einzusetzen.

Die Reaktionstemperaturen können bei der Durchführung der gegebenenfalls durchzuführenden 4. Stufe des erfindungsgemäßen Verfahrens (Amidierungsreaktion) in einem größeren Bereich variiert werden. Im allgemeinen arbeitet man bei Temperaturen von -30 °C bis +150 °C, vorzugsweise bei Temperaturen von -20 °C bis +120 °C.

Die gegebenenfalls durchzuführende 4. Stufe des erfindungsgemäßen Verfahrens (Amidierungsreaktion) wird üblicherweise unter Normaldruck durchgeführt. Es ist jedoch auch möglich unter erhöhtem Druck beispielsweise zwischen 1 und 100 bar zu arbeiten

Zur Durchführung der gegebenenfalls durchzuführenden 4. Stufe des erfindungsgemäßen Verfahrens (Amidierungsreaktion) setzt man pro Mol an 2-Aryl-2,2-dialkoxyessigsäurederivate der Formel (IVa) bzw. (VIa) bzw. pro Mol an 2-Oximinoessigsäurederivat der Formel (Ia) im allgemeinen 1,0 bis 3,0 Mol, vorzugsweise 1,0 bis 1,5 Mol an Amin der Formel (VIII) und gegebenenfalls 0,1 bis 3,0 Mol, vorzugsweise 0,5 bis 1,5 Mol an als Reaktionshilfsmittel verwendeter Base ein. Wird die Amidierungsreaktion zwischen 1. und 2. Stufe des erfindungsgemäßen Verfahren eingeschoben, verwendet man 2-Aryl-2,2-dialkoxy-essigsäurederivate der Formel (IVa) als Ausgangverbindungen, wird die Amidierungsreaktion zwischen 2. und 3. Stufe des erfindungsgemäßen Verfahren eingeschoben, verwendet man 2-Aryl-2,2-dialkoxyessigsäurederivate der Formel (VIa) als Ausgangverbindungen, wird die Amidierungsreaktion nach der Durchführung der 3. Stufe des erfindungsgemäßen Verfahrens durchgeführt, verwendet man 2-Oximinoessigsäurederivate der Formel (Ia) als Ausgangverbindungen. Die Reaktionsdurchführung, Aufarbeitung und Isolierung der Reaktionsprodukte erfolgt jeweils in Analogie zu bekannten Verfahren.

Die Reinigung der Produkte erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1H$-NMR).

Die erfindungsgemäß herstellbaren Verbindungen der Formel (I) sind bekannte Fungizide (vergl. z.B. EP 398 692 und EP 468 775).

Herstellungsbeispiele:

Beispiel IV-1:

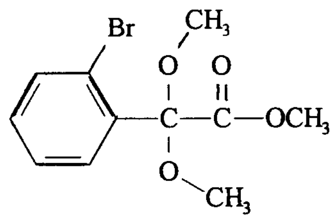

(1. Stufe)

Zu 44,8 g (0,226 Mol) 2-Bromphenylglyoxylsäuremethylester und 239 g (2,26 Mol) Orthoameisensäuretrimethylester in 100 ml Methanol gibt man 2 ml konzentrierte Schwefelsäure und erhitzt für 24 Stunden auf Rückflußtemperatur. Zur Aufarbeitung wird die Reaktionsmischung abgekühlt 10 ml gesättigte, wässrige Natriumhydrogencarbonatlösung zugegeben, anschließend im Vakuum eingeengt, der Rückstand mit Essigester aufgenommen, mit Wasser gewaschen, über Natriumsulfat getrocknet, im Vakuum eingeengt und im Hochvakuum destilliert.

Man erhält 2,2-Dimethoxy-2-(2-bromphenyl)-essigsäuremethylester (Ausbeute 85 % der Theorie; Schmelzpunkt 55 ° C).

Beispiel VI-1:

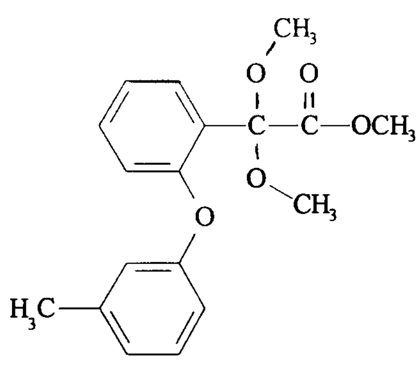

(2. Stufe)

Zu einer Lösung von 5,4 g (0,1 Mol) Natriummethylat in 100 ml Methanol gibt man 10,1 g (0,1 mol) 3-Methylphenol und engt zur Trockne ein. Der Rückstand wird mit 3,23 g (0,01 Mol) Tris-[2-(2-methoxyethoxy)-ethyl]-amin, 1 g (0,01 Mol) Kupfer-(I)-chlorid, 60 ml Diethylenglykoldimethylether und 28,9 g (0,1 Mol) 2,2-Dimethoxy-2-(2-bromphenyl)-essigsäuremethylester versetzt und unter Rühren für 48 Stunden auf 165 ° C erhitzt. Zur Aufarbeitung wird die Reaktionsmischung abgekühlt, mit Wasser versetzt, mit Dichlormethan extrahiert, die organische. Phase getrocknet, im Vakuum eingeengt und der Rückstand über Kieselgel chromatographiert (Laufmittel: Cyclohexan/Essigester 3:1)

Man erhält 2,2-Dimethoxy-2-[2-(3-methylphenoxy)-phenyl]-essigsäuremethylester als Öl (Ausbeute 75 % der Theorie).

$^1$H-NMR ($CDCl_3$/Tetramethylsilan): $\delta$ = 2,3 (3H); 3,2 (6H); 3,6 (3H); 6,65-7,9 (8H) ppm.

Beispiel I-1:

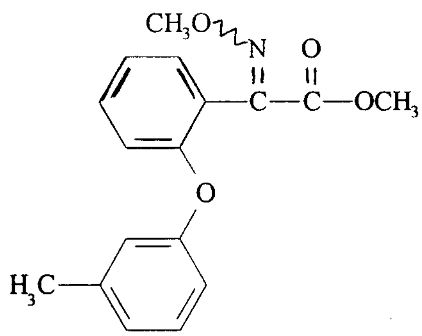

(3. Stufe)

Zu 3,16 g (0,01 Mol) 2,2-Dimethoxy-2-[2-(3-methylphenoxy)-phenyl]-essigsäuremethylester und 1,5 g (0,0175 Mol) O-Methylhydroxylaminhydrochlorid in 100 ml Methanol gibt man 1 ml konzentrierte Salzsäure und erhitz für 18 Stunden auf Rückflußtemperatur. Zur Aufarbeitung wird die Reaktionsmischung eingeengt, der Rückstand in Essigester aufgenommen, mit gesättigter, wässriger Natriumhydrogencarbonatlösung gewaschen, über Natriumsulfat getrocknet, im Vakuum eingeengt und der Rückstand über Kieselgel chromatographiert (Laufmittel: Cyclohexan/Essigester 3:1)
Man erhält 2-Methoximino-2-[2-(3-methylphenoxy)-phenyl]-essigsäuremethylester als Öl (Ausbeute 70% der Theorie).

Beispiel I-2:

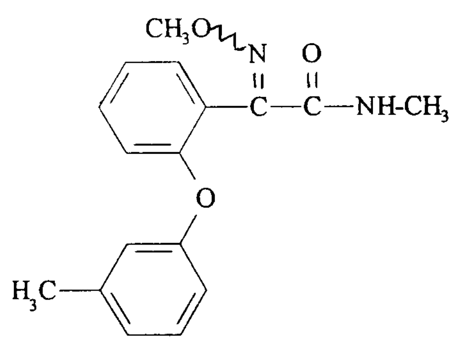

(4. Stufe / Amidierungsreaktion)

In eine Lösung von 2,99 g (0,01 Mol) 2-Methoximino-2-[2-(3-methylphenoxy)-phenyl]-essigsäuremethylester in 200 ml Methanol leitet man unter Eiskühlung bis zur Sättigung Methylamingas ein und rührt die Reaktionsmischung anschließend 18 Stunden bei 20 °C. Zur Aufarbeitung wird Mischung eingeengt und der Rückstand aus Ethanol unkristallisiert.

Man erhält 2-Methoximino-2-[2-(3-methylphenoxy)-phenyl]-essigsäure-N-methylamid (Ausbeute 70 % der Theorie).

In entsprechender Weise und gemäß den allgemeinen Angaben zur Herstellung erhält man die folgenden 2-Aryl-2,2-dialkoxy-essigsäurederivate der allgemeinen Formel (IX):

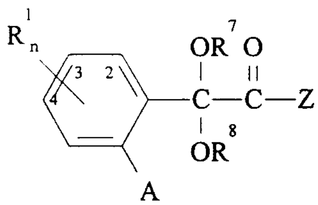

(IX)

| Bsp. Nr. | A | $R^1$ | $R^7$ | $R^8$ | n | Z | physikalische Eigenschaften |
|---|---|---|---|---|---|---|---|
| IV-2 | Cl | H | $CH_3$ | $CH_3$ | 0 | $-O-CH_3$ | Fp. 73-76°C |
| VI-2 | 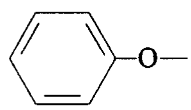 | H | $CH_3$ | $CH_3$ | 1 | $-O-CH_3$ | $^1$H-NMR*): 3,2 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Patentansprüche

1. Verfahren zur Herstellung von 2-Oximinoessigsäurederivaten der allgemeinen Formel (I),

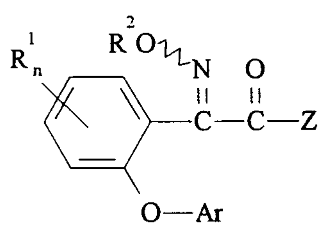

(I)

in welcher

| | |
|---|---|
| $R^1$ | für Alkyl oder Alkoxy steht, |
| $R^2$ | für Alkyl steht, |
| Ar | für gegebenenfalls substituiertes Aryl oder Heteroaryl steht, |
| Z | für einen Rest der Formel $-O-R^3$ oder $-NR^4R^5$ steht und |
| n | für eine Zahl 0, 1, 2, 3 oder 4 steht, wobei |
| $R^3$ | für Alkyl steht und |
| $R^4$ und $R^5$ | unabhängig voneinander jeweils für Wasserstoff, Alkyl, Halogenalkyl oder Alkoxy stehen, |

dadurch gekennzeichnet, daß man 2-Oxo-2-phenyl-essigäurederivate der Formel (II),

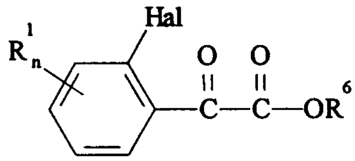

(II)

,

in welcher

Hal für Halogen steht,

$R^6$ für Wasserstoff oder Alkyl steht und

$R^1$ und n die oben angegebene Bedeutung haben,

zunächst in einer 1. Stufe mit Alkoholen der Formel (IIIa),

$R^7$-OH (IIIa)

in welcher

$R^7$ für Alkyl steht,

oder mit Diolen der Formel (IIIb),

HO-$R^7$-$R^8$-OH (IIIb)

in welcher

$R^7$ und $R^8$ gemeinsam für einen zweifach verknüpften Alkandiylrest stehen,

gegebenenfalls in Gegenwart eines Verdünnungsmittels und gegebenenfalls in Gegenwart eines Reaktionshilfsmittels umsetzt und in einer anschließenden 2. Stufe die so erhältlichen 2-Aryl-2,2-dialkoxy-essigsäurederivate der Formel (IVa),

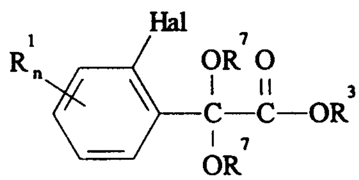

(IVa-1)

bzw. 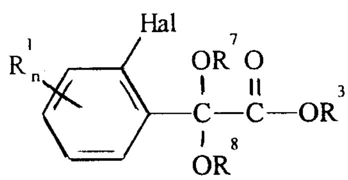 (IVa-2)

in welcher

$R^1$, $R^3$, $R^7$, $R^8$, Hal und n die oben angegebene Bedeutung haben,

mit Hydroxyverbindungen der Formel (V),

Ar-OH (V)

in welcher

Ar die oben angegebene Bedeutung hat,

gegebenenfalls in Gegenwart eines Verdünnungsmittels und gegebenenfalls in Gegenwart eines Reaktionshilfsmittels umsetzt und anschließend die so erhältlichen 2-Aryl-2,2-dialkoxy-essigsäurederivate der Formel (VIa),

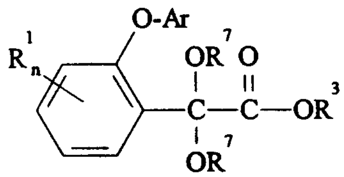

(VIa-1)

bzw. 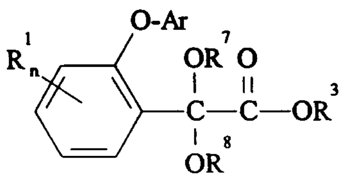

(VIa-2)

in welcher

$R^1$, $R^3$, $R^7$, $R^8$, Ar und n die oben angegebene Bedeutung haben,

in einer darauffolgenden 3. Stufe mit Hydroxylaminderivaten der Formel (VII),

$R^2$-O-$NH_2$ (VII)

in welcher

$R^2$ die oben angegebene Bedeutung hat,

oder mit deren Hydrohalogenidsalzen gegebenenfalls in Gegenwart eines Verdünnungsmittels und gegebenenfalls in Gegenwart eines Reaktionshilfsmittels zu den 2-Oximinoessigsäurederivaten der Formel (Ia),

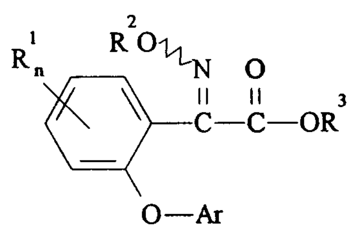

(Ia)

in welcher

$R^1$, $R^2$, $R^3$, Ar und n die oben angegebene Bedeutung haben,

umsetzt, wobei die in der ersten Stufe erhältlichen 2-Aryl-2,2-dialkoxy-essigsäurederivate der Formel (IVa) oder die in der zweiten Stufe erhältlichen 2-Aryl-2,2-dialkoxy-essigsäurederivate der Formel (VIa) oder die in der dritten Stufe erhältlichen 2-Oximinoessigsäurederivaten der Formel (Ia) gegebenenfalls in einer zwischengeschalteten Reaktion bzw. in einer Folgereaktion jeweils mit Aminen der Formel (VIII),

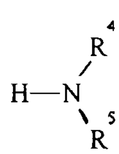

(VIII)

in welcher

$R^5$ und $R^6$ die oben angegebene Bedeutung haben,

gegebenenfalls in Gegenwart eines Verdünnungsmittels und gegebenenfalls in Gegenwart eines Reaktionshilfsmittels umgesetzt werden.

**2.** Verfahren gemäß Anspruch 1, worin

$R^1$ für geradkettiges oder verzweigtes Alkyl mit 1 bis 6 Kohlenstoffatomen oder für geradkettiges oder verzweigtes Alkoxy mit 1 bis 6 Kohlenstoffatomen steht,

$R^2$ für geradkettiges oder verzweigtes Alkyl mit 1 bis 6 Kohlenstoffatomen steht.

Ar für jeweils gegebenenfalls einfach oder mehrfach, gleich oder verschieden substituiertes Aryl mit 6 bis 10 Kohlenstoffatomen oder Heteroaryl mit 2 bis 9 Kohlenstoffatomen und 1 bis 4 gleichen oder verschiedenen Heteroatomen - insbesondere Stickstoff Sauerstoff und/oder Schwefel - steht, wobei als Substituenten jeweils infrage kommen: Halogen, Cyano, Nitro, jeweils geradkettiges oder verzweigtes Alkyl, Alkoxy, Alkylthio mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Alkinyl oder Alkinyloxy mit jeweils 2 bis 6 Kohlenstoffatomen, jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Halogenalkinyl oder Halogenalkinyloxy mit jeweils 2 bis 6 Kohlenstoffatomen und 1 bis 13 gleichen oder verschiedenen Halogenatomen, jeweils geradkettiges oder verzweigtes Alkoxycarbonyl, Hydroximinoalkyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - substituiertes zweifach verknüpftes Dioxyalkylen mit 1 bis 5 Kohlenstoffatomen oder jeweils gegebenenfalls im Phenylteil einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Benzyl, Benzyloxy, Phenylethyl oder Phenylethyloxy,

Z für einen Rest der Formel $-O-R^3$ oder $-NR^4R^5$ steht und

n für eine Zahl 0, 1, 2, 3 oder 4 steht, wobei

$R^3$ für geradkettiges oder verzweigtes Alkyl mit 1 bis 6 Kohlenstoffatomen steht und

$R^4$ und $R^5$ unabhängig voneinander jeweils für Wasserstoff für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen steht oder für geradkettiges oder verzweigtes Alkoxy mit 1 bis 8 Kohlenstoffatomen stehen.

**3.** Verfahren gemäß Anspruch 1, worin

$R^1$ für geradkettiges oder verzweigtes Alkyl mit 1 bis 4 Kohlenstoffatomen oder für geradkettiges oder verzweigtes Alkoxy mit 1 bis 4 Kohlenstoffatomen steht,

$R^2$ für geradkettiges oder verzweigtes Alkyl mit 1 bis 4 Kohlenstoffatomen steht,

Ar für jeweils gegebenenfalls einfach bis fünffach, gleich oder verschieden substituiertes Aryl mit 6 oder 10 Kohlenstoffatomen oder Heteroaryl mit 2 bis 9 Kohlenstoffatomen und 1 bis 3 gleichen oder verschiedenen Heteroatomen - insbesondere Stickstoff Sauerstoff und/oder Schwefel - steht, wobei als Substituenten jeweils infrage kommen: Halogen, Cyano, Nitro, jeweils geradkettiges oder verzweigtes Alkyl, Alkoxy, Alkylthio mit jeweils 1 bis 4 Kohlenstoffatomen, jeweils geradkettiges oder verzweigtes Alkenyl oder Alkenyloxy mit jeweils 2 bis 5 Kohlenstoffatomen, jeweils geradkettiges oder verzweigtes Alkinyl oder Alkinyloxy mit jeweils 2 bis 5 Kohlenstoffatomen, jeweils

geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Halogenalkenyl oder Halogenalkenyloxy mit jeweils 2 bis 5 Kohlenstoffatomen und 1 bis 5 gleichen oder verschiedenen Halogenatomen, jeweils geradkettiges oder verzweigtes Halogenalkinyl oder Halogenalkinyloxy mit jeweils 2 bis 5 Kohlenstoffatomen und 1 bis 5 gleichen oder verschiedenen Halogenatomen, jeweils geradkettiges oder verzweigtes Alkoxycarbonyl, Hydroximinoalkyl oder Alkoximinoalkyl mit jeweils 1 bis 4 Kohlenstoffatomen in den einzelnen Alkylteilen, gegebenenfalls einfach bis vierfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - substituiertes zweifach verknüpftes Dioxyalkylen mit 1 bis 3 Kohlenstoffatomen oder jeweils gegebenenfalls im Phenylteil einfach bis fünffach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 3 Kohlenstoffatomen und 1 bis 7 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 3 Kohlenstoffatomen und 1 bis 7 gleichen oder verschiedenen Halogenatomen substituiertes Phenyl, Phenoxy, Benzyl, Benzyloxy, Phenylethyl oder Phenylethyloxy,

Z für einen Rest der Formel $-O-R^3$ oder $-NR^4R^5$ steht und
n für eine zahl 0, 1, 2 oder 3 steht, wobei
$R^3$ für geradkettiges oder verzweigtes Alkyl mit 1 bis 4 Kohlenstoffatomen steht und
$R^4$ und $R^5$ unabhängig voneinander jeweils für Wasserstoff für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen steht oder für geradkettiges oder verzweigtes Alkoxy mit 1 bis 6 Kohlenstoffatomen stehen.

**4.** Verfahren gemäß Anspruch 1, worin

$R^1$ für Methyl, Methoxy, Ethyl oder Ethoxy steht,
$R^2$ für Methyl oder Ethyl steht,
Ar für gegebenenfalls einfach bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Allyl, Butenyl, Allyloxy, Butenyloxy, Trifluormethyl, Trifluormethoxy, Difluormethoxy, Trifluormethylthio, Difluorchlormethylthio, Methoxycarbonyl, Ethoxycarbonyl, Hydroximinomethyl, Hydroximinoethyl, Methoximinomethyl, Methoximinoethyl, Ethoximinomethyl, Ethoximinoethy, gegebenenfalls einfach bis vierfach, gleich oder verschieden durch Fluor, Chlor, Brom, Methyl, Ethyl und/oder Trifluormethyl substituiertes zweifach verknüpftes Dioxymethylen oder Dioxyethylen oder jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy,
Z für einen Rest der Formel $-O-R^3$ oder $-NR^4R^5$ steht und
n für eine Zahl 0,1, oder 2 steht, wobei
$R^3$ für Methyl oder Ethyl steht und
$R^4$ und $R^5$ unabhängig voneinander jeweils für Wasserstoff für geradkettiges oder verzweigtes Alkyl mit 1 bis 4 Kohlenstoffatomen, für Halogenalkyl mit 1 bis 4 Kohlenstoffatomen und 1 bis 5 gleichen oder verschiedenen Halogenatomen steht oder für geradkettiges oder verzweigtes Alkoxy mit 1 bis 4 Kohlenstoffatomen stehen.

**5.** 2-Aryl-2,2-dialkoxy-essigsäurederivate der Formel (IX),

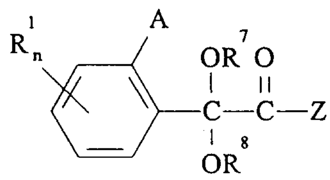

(IX)

in welcher

| | |
|---|---|
| $R^1$ | für Alkyl oder Alkoxy steht, |
| $R^7$ und $R^8$ | entweder gleich sind und jeweils für Alkyl stehen oder gemeinsam für einen zweifach verknüpften Alkandiylrest stehen, |
| A | für Halogen oder für gegebenenfalls substituiertes Aryloxy oder Heteroaryloxy steht, |
| Z | für einen Rest der Formel -O-$R^3$ oder -$NR^4R^5$ steht und |
| n | für eine Zahl 0, 1, 2, 3 oder 4 steht, wobei |
| $R^3$ | für Alkyl steht und |
| $R^4$ und $R^5$ | unabhängig voneinander jeweils für Wasserstoff Alkyl, Halogenalkyl oder Alkoxy stehen. |

**6.** Verbindungen der Formel (IX) gemäß Anspruch 5, bei welchen

| | |
|---|---|
| $R^1$ | für geradkettiges oder verzweigtes Alkyl mit 1 bis 6 Kohlenstoffatomen oder für geradkettiges oder verzweigtes Alkoxy mit 1 bis 6 Kohlenstoffatomen steht, |
| $R^7$ und $R^8$ | entweder gleich sind und für geradkettiges oder verzweigtes Alkyl mit 1 bis 6 Kohlenstoffatomen stehen oder gemeinsam für einen zweifach verknüpften Alkandiylrest mit 1 bis 6 Kohlenstoffatomen stehen, |
| A | für Fluor, Chlor, Brom, Iod oder für jeweils gegebenenfalls einfach oder mehrfach, gleich oder verschieden substituiertes Aryloxy mit 6 bis 10 Kohlenstoffatomen oder Heteroaryloxy mit 2 bis 9 Kohlenstoffatomen und 1 bis 4 gleichen oder verschiedenen Heteroatomen - insbesondere Stickstoff Sauerstoff und/oder Schwefel - steht, wobei als Substituenten jeweils infrage kommen: Halogen Cyano, Nitro, jeweils geradkettiges oder verzweigtes Alkyl, Alkoxy, Alkylthio mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Alkinyl oder Alkinyloxy mit jeweils 2 bis 6 Kohlenstoffatomen, jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Halogenalkinyl oder Halogenalkinyloxy mit jeweils 2 bis 6 Kohlenstoffatomen und 1 bis 13 gleichen oder verschiedenen Halogenatomen, jeweils geradkettiges oder verzweigtes Alkoxycarbonyl, Hydroximinoalkyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - substituiertes zweifach verknüpftes Dioxyalkylen mit 1 bis 5 Kohlenstoffatomen oder jeweils gegebenenfalls im Phenylteil einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Benzyl, Benzyloxy, Phenylethyl oder Phenylethyloxy, |
| Z | für einen Rest der Formel -O-$R^3$ oder -$NR^4R^5$ steht und |
| n | für eine Zahl 0, 1, 2, 3 oder 4 steht, wobei |

| | |
|---|---|
| $R^3$ | für geradkettiges oder verzweigtes Alkyl mit 1 bis 6 Kohlenstoffatomen steht und |
| $R^4$ und $R^5$ | unabhängig voneinander jeweils für Wasserstoff für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen steht oder für geradkettiges oder verzweigtes Alkoxy mit 1 bis 8 Kohlenstoffatomen stehen. |

**7.** Verwendung der Verbindungen der Formel (IX) nach den Ansprüchen 5 und 6 zur Herstellung von 2-Oximinoessigsäurederivaten der Formel (I) nach den Ansprüchen 1 bis 4.